(19) Europäisches Patentamt
European Patent Office
Office européen des brevets

(11) Publication number: 0 169 658 A1

# (12) EUROPEAN PATENT APPLICATION

(21) Application number: 85304326.3

(22) Date of filing: 17.06.85

(51) Int. Cl.⁴: A 61 K 47/00

(30) Priority: 18.06.84 US 621580

(43) Date of publication of application: 29.01.86 Bulletin 86/5

(84) Designated Contracting States: AT BE CH DE FR GB IT LI LU NL SE

(71) Applicant: SCHERING CORPORATION
2000 Galloping Hill Road
Kenilworth, New Jersey 07033(US)

(72) Inventor: Shah, Mahendra
106 Longview Avenue
Lake Hiawatha New Jersey 07034(US)

(74) Representative: Ritter, Stephen David et al,
Mathys & Squire 10 Fleet Street
London EC4Y 1AY(GB)

**(54) Substained release dosage form.**

(57) Sustained release pharmaceutical dosage forms are made of saponified starch-acrylonitrile graft copolymers and an active ingredient.

EP 0 169 658 A1

Case 2338

SUSTAINED RELEASE DOSAGE FORM

This invention relates to improved drug delivery systems.

Sustained or controlled release systems for drug delivery have been developed in response to the shortcomings of conventional drug forms. When a conventional tablet or capsule is taken, the concentration of the drug in the blood stream may rise rapidly to a level that is higher than the so-called "therapeutic range" (the range in which the blood concentration of the drug is sufficient to produce a desired therapeutic effect). If the concentration of the drug is below this range, the drug is ineffective. If it is above this range, the drug is toxic in the sense that it may cause side effects. Delivery systems which release the drug to the body in a controlled manner over a sustained period of time have the advantage of raising the concentration of the therapeutic agent in the blood stream to the "therapeutic range" and maintaining the active agent within that range over a prolonged period of time. This presents the drug to the body at an effective

level over an extended period of time without causing any toxic side affects.

Early sustained release systems included highly compressed tablets, drugs formulated in suspensions or emulsions, drug-ion exchange resin complexes and low solubility salt forms of active substances. Such methods, however, generally did not produce a sufficiently slow release rate, nor did they produce a sufficiently well controlled release rate.

More recent systems utilize the principles of diffusion, chemical reaction or solvent activation to extend the release rate of the drug. Diffusion systems may utilize a reservoir or a non-erodible matrix. In reservoir systems there is a core of active material surrounded by a non-degradable polymer (the rate limiting material) through which the active material moves by diffusion. In non-erodible matrix systems the active ingredient is uniformly distributed throughout a polymer matrix.

Systems which utilize chemical reaction to provide a sustained release system utilize erodible matrices in which erosion takes place by the hydrolysis of a polymer backbone or through erosion of cross linkages or pendent groups on the polymer. Yet another class of systems are activated by solvents in their environment. Some systems utilize more than one of these principles, such as those which utilize a combination of diffusion and erosion. The present system utilizes all three modes of action. It comprises a solvent (water) activated system which, upon activation, utilizes diffusion and a slow erosion by chemical reaction to slowly release a physiologically or pharmacologically active substance over an extended period of time and in a controlled

manner. The rate limiting component of the present system is a saponified starch-acrylonitrile graft copolymer. Saponified starch-acrylonitrile graft copolymers suitable for use in the present system are disclosed in U.S. Patent 3.661,815 to Smith dated May 9, 1972; U.S. Patent 4,116,899 to Funta et al. dated September 26, 1978; and U.S. Patent 4,159,260 to Jones et al. dated June 26, 1979. These references disclose compositions of alkaline metal carboxylate salts of starch-polyacrylonitrile graft copolymers which are prepared by saponifying the starch-polyacrylonitrile graft copolymers with an aqueous alcoholic solution of an alkali metal base. These products are water insoluble granular solids having the ability to absorb water in amounts in excess of fifty parts per part while maintaining their granular character.

Graft copolymers of starch-polyacrylonitrile per se as well as methods for their preparation are known. Acrylonitrile can be grafted onto starch using ceric salts as catalysts to form starch-acrylonitrile graft copolymers. (Such systems are disclosed in U.S. Patent 2,922,768.) Graft copolymers can also be prepared by the reaction of acrylonitrile with preirradiated starch which is prepared by irradiation of starch with gamma rays or an electron beam. In such graft copolymers the starch serves as a backbone or building block on which the acrylonitrile is grafted. The starch therefore need be present in only very small proportions with respect to the polyacrylonitrile moiety. Starch-polyacrylonitrile graft copolymer starting materials can be prepared by any of the known methods and the ratio of acrylonitrile to starch can be exceedingly high. In general, however, the molar ratio of starch to acrylonitrile in the starting graft

copolymers should be at least 1:2 and generally in the range of 1:3 to 1:30.

Saponification of starch-polyacrylonitrile graft copolymers with aqueous solutions of bases such as potassium hydroxide is also known. Saponification of starch-polyacrylonitrile or starch-polymethacrylonitrile graft copolymers with an aqueous alcoholic solution of a base provides products having particularly useful characteristics.

Blending a hydrolysed polyacrylonitrile-starch graft copolymer with a fatty quaternary ammonium chloride containing at least 18 carbon atoms allows the resulting material to disperse in water without agitation. This blend is described in U.S. Patent 4,159,260 and is sold by Henkel Corporation, Minneapolis, Minnesota under the grade designation SGP 104.

An example of a particularly suitable polymer for use in the present invention is that sold by Henkel Corporation, Minneapolis, Minnesota under the grade designation SGP 502S. This polymer is a graft terpolymer of starch, acrylamide and sodium acrylate whose structure is, in part, as follows:

FIGURE 1*

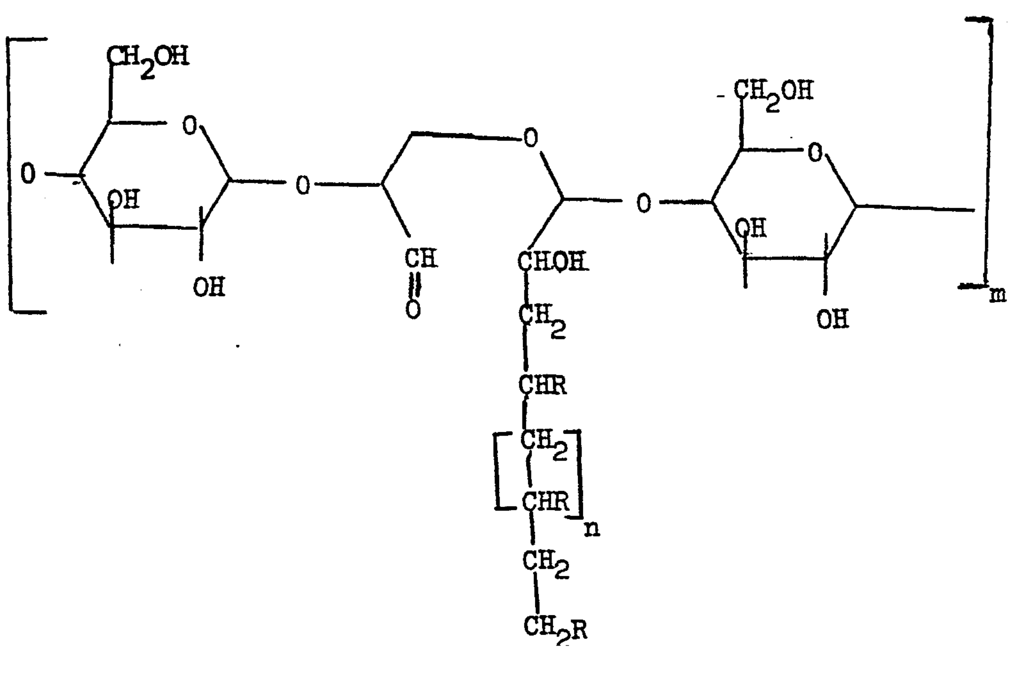

where R= $CONH_2$ or $CO_2Na$
*grafting may also occur at $C_3$

This particular polymer is produced by a two step reaction. In the first step, acrylonitrile is polymerized in the presence of starch using ceric (IV) catalyst to initiate polymerization. Ceric ion coordinates with starch and effects polymerization of acrylonitrile onto starch. The product is a graft polymer of starch and acrylonitrile and is designated as starch-g-poly(acrylonitrile).

In the second step, starch-g-poly(acrylonitrile) is hydrolysed with strong alkali. This converts nitrile groups in the poly(acrylonitrile) portion of the graft polymer (and in unreacted acrylonitrile monomer) to carboxamide and carboxylate groups (amides are the primary product of nitrile hydrolysis; carboxylates are the secondary product).

The polymer is precipitated from the reaction mixture by the addition of methanol, repetitively extracted with methanol to remove reaction by-product and contaminants, filtered and dried.

The polymer and physiologically or pharmacologically active substances can be combined in various types of dosage forms. The combination product may be used to prepare oral solutions, syrups and elixirs; oral suspensions, emulsions, magmas and gels; powders, granules and solid dosage forms for oral administration such as tablets and capsules; injectables; ointments, creams, lotions or other dermatologic preparations; ophthalmic preparations; ear, nose, or topical oral preparations; or as a suppository or other rectal, vaginal or urethral preparations.

The ratio of active ingredient to polymer

will depend upon the dosage form and the period of release desired. In general, the active ingredient should comprise from about 30 to about 90% by weight of the composition. It is desirable to maintain the percentage of active ingredient at as high a level as possible consistent with maintenance of an extended release period.

The polymer is combined with a drug or physiologically or pharmacologically active substance appropriate for the required purpose. As used herein the terms "drug" and "physiologically or pharmacologically active substance" are used interchangeably to identify physiologically or pharmacologically active substances that produce a local or systemic therapeutic effect in animals, including warm-blooded mammals, humans and primates, domestic, household, sport, farm, zoo, laboratory or wild animals such as birds, sheep, goats, cattle, horses, pigs, mice, rats and fish.

The drug can be an organic or inorganic compound and may be in various chemical or physical forms such as uncharged molecules, molecular complexes or pharmacologically acceptable acid and base addition salts. The drug may be delivered in a biologically active form or in a form such that it becomes active only after conversion to a biologically active species in the body of the animal such as by enzyme conversion, hydrolysis or other metabolic process.

The drug may be in the form of a finely divided powder, liquid, dispersion, gel, paste, cream, granule, particle, emulsion or suspension.

The drug may comprise a combination of more than one active substance or may be a combination of one or more active substances combined with binders,

dispersants, emulsifiers, wetting agents, or coloring agents.

The classes of drugs which may be used include both local and systemically active substances such as gastrointestinal drugs; blood, fluid, electrolyte and hematologic drugs, cardiovascular drugs, respiratory drugs; sympathomimetic drugs; parasympathomimetic drugs; adrenergic blocking drugs, antimuscarinic and antispasmodic drugs; skeletal muscle relaxants; diuretics; uterine and antimigraine drugs, hormones, vitamins and other nutrients; anesthetics; sedatives and hypnotics; antiepileptics; psychopharmacologic agents; analgesics and antipyretics; antihistamines; central nervous systems stimulants; antineoplastic and immunosuppressive drugs; antimicrobial drugs; parasiticides; pesticides and diagnostic drugs. Specific examples of these drugs and typical dosage amounts can be found in any of the standard text, as for example, Remington's Pharmaceutical Sciences, 16th edition, 1980, published by the Mack Publishing Co., Easton, PA, U.S.A.

To make sustained dosage forms in accordance with the invention, saponified starch-acrylonitrile graft copolymer is mixed with the active ingredient and the mixture is then placed into the desired form. For example, to form a tablet, compress the mixture in a conventional tablet press. To form a suspension for injection or oral administration, mix the mixture with appropriate liquids.

## EXAMPLES

The following examples illustrate the effect of saponified starch-acrylonitrile graft copolymer on rate of the slow release of various biologically active substances. Two graft copolymers were used: (1) SGP 502S, manufactured by Henkel Corporation, and (2) SGP 104, which is made by blending SGP 502S with a fatty quaternary ammonium chloride.

For all examples, tablets were made by compressing 250 mg of a mixture of active substance and graft copolymer at 4000 lb pressure on a Carver Tablet press using 11/36" standard concave punches.

To test the release properties of the tablet, simulated gastric fluid (SGF) and simulated intestinal fluid (SIF) were prepared according to United States Pharmacopoeia XX. The dissolution rates of the active substances were determined at various times at 37°C.

### EXAMPLES WITH SALICYLIC ACID AS THE ACTIVE INGREDIENT

| Example No. | | 1 | 2 | 3 | 4 |
|---|---|---|---|---|---|
| Wt. % SGP 104 | | 0 | 30 | 50 | 70 |
| Wt. % Salicylic Acid | | 100 | 70 | 50 | 30 |
| Time, Hrs | Fluid | % Salicylic Acid Dissolved | | | |
| 1 | SGF | 13 | 48 | 12 | 14 |
| 2 | SIF | 45 | 87 | 21 | 30 |
| 3 | SIF | 68 | | 32 | 49 |
| 4 | SIF | 83 | | 44 | 69 |
| 5 | SIF | 97 | | 57 | 82 |
| 6 | SIF | | | 65 | 90 |
| 18 | SIF | | | 103 | 101 |

The above examples show how the dissolution rate of salicylic acid can be varied with varying concentrations of the graft copolymer.

| Example No. | | 5 | 6 | 7 | 8 |
|---|---|---|---|---|---|
| Wt. % SGP 502S | | 0 | 30 | 50 | 70 |
| Wt. % Salicylic Acid | | 100 | 70 | 50 | 30 |
| Time, Hrs | Fluid | % Salicylic Acid Dissolved | | | |
| 1 | SGF | 13 | 8 | 10 | 18 |
| 2 | SIF | 45 | 18 | 23 | 39 |
| 3 | SIF | 68 | 28 | 40 | 65 |
| 4 | SIF | 83 | 37 | 56 | 80 |
| 5 | SIF | 97 | 43 | 65 | 103 |
| 6 | SIF | | 49 | 72 | |
| 24 | SIF | | 102 | 100 | |

EXAMPLES WITH SODIUM SALICYLATE
AS THE ACTIVE INGREDIENT

| Example No. | | 9 | 10 | 11 | 12 |
|---|---|---|---|---|---|
| Wt. % SGP 104 | | 0 | 30 | 50 | 70 |
| Wt. % Sodium Salicylate | | 100 | 70 | 50 | 30 |
| Time, Hrs | Fluid | % Sodium Salicylate Dissolved | | | |
| 1 | SGF | 100 | 102 | 28 | 24 |
| 2 | SIF | | | 57 | 47 |
| 3 | SIF | | | 77 | 67 |
| 4 | SIF | | | 84 | 78 |
| 5 | SIF | | | 89 | 86 |

| Example No. | | 13 | 14 | 15 | 16 |
|---|---|---|---|---|---|
| Wt. % SGP 502S | | 0 | 30 | 50 | 70 |
| Wt. % Sodium Salicylate | | 100 | 70 | 50 | 30 |
| Time, Hrs | Fluid | % Sodium Salicylate Dissolved | | | |
| 1 | SGF | 100 | 102 | 32 | 28 |
| 2 | SIF | | | 71 | 63 |
| 3 | SIF | | | 84 | 79 |
| 4 | SIF | | | 92 | 89 |
| 5 | SIF | | | 95 | 94 |

Examples 9 through 16 show how a very rapidly dissolving active ingredient can be slowly released using the present invention.

## EXAMPLES WITH PSEUDOEPHEDRINE BASE (PB) AS THE ACTIVE INGREDIENT

| Example No. | | 17 | 18 | 19 | 20 | 21 |
|---|---|---|---|---|---|---|
| Wt. % SGP 104 | | 0 | 30 | 50 | 60 | 70 |
| Wt. % PB | | 100 | 70 | 50 | 40 | 30 |
| Time, Hrs | Fluid | % PB Dissolved | | | | |
| 1 | SGF | 100 | 63 | 25 | 21 | 24 |
| 2 | SIF | | 100 | 62 | 30 | 32 |
| 3 | SIF | | | 87 | 45 | 43 |
| 4 | SIF | | | 95 | 57 | 64 |
| 5 | SIF | | | | 70 | 78 |
| 6 | SIF | | | | 83 | 93 |
| 7 | SIF | | | | 89 | |

| Example No. | | 22 | 23 | 24 | 25 | 26 |
|---|---|---|---|---|---|---|
| Wt. % SGP 502S | | 0 | 30 | 50 | 60 | 70 |
| Wt. % PB | | 100 | 70 | 50 | 40 | 30 |
| Time, Hrs | Fluid | % PB Dissolved | | | | |
| 1 | SGF | 100 | 64 | 25 | 21 | 22 |
| 2 | SIF | | 95 | 66 | 30 | 28 |
| 3 | SIF | | | 90 | 45 | 34 |
| 4 | SIF | | | 96 | 57 | 40 |
| 5 | SIF | | | | 70 | 58 |
| 6 | SIF | | | | 83 | 59 |
| 7 | SIF | | | | 89 | |

Example 17 to 26 also illustrate the sustained release of a rapidly dissolving ingredient.

EXAMPLES WITH PSEUDOEPHEDRINE SULFATE (PS) AS THE ACTIVE INGREDIENT

| Example No. | | 27 | 28 | 29 | 30 | 31 |
|---|---|---|---|---|---|---|
| Wt. % SGP 104 | | 0 | 30 | 40 | 50 | 70 |
| Wt. % PS | | 100 | 70 | 60 | 50 | 30 |
| Time, Hrs | Fluid | % PS Dissolved | | | | |
| 1 | SGF | 100 | 94 | 45 | 39 | 33 |
| 2 | SIF | | | 63 | 52 | 40 |
| 3 | SIF | | | 69 | 59 | 45 |
| 4 | SIF | | | 79 | 66 | 52 |
| 5 | SIF | | | 82 | 69 | 58 |
| 6 | SIF | | | 87 | 76 | 65 |
| 7 | SIF | | | 89 | | |

| Example No. | | 32 | 33 | 34 | 35 | 36 |
|---|---|---|---|---|---|---|
| Wt. % SGP 502S | | 0 | 30 | 40 | 50 | 70 |
| Wt. % PS | | 100 | 70 | 60 | 50 | 30 |
| Time, Hrs | Fluid | % PS Dissolved | | | | |
| 1 | SGF | 100 | 56 | 30 | 24 | 25 |
| 2 | SIF | | 76 | 41 | 33 | 32 |
| 3 | SIF | | 87 | 47 | 38 | 38 |
| 4 | SIF | | | 52 | 44 | 45 |
| 5 | SIF | | | 59 | 48 | 46 |
| 6 | SIF | | | 62 | 52 | 51 |
| 7 | SIF | | | 67 | 57 | 55 |

## EXAMPLES WITH CHLORAMPHENICOL (C) AS THE ACTIVE INGREDIENT

| Example No. | | 37 | 38 | 39 | 40 |
|---|---|---|---|---|---|
| Wt. % SGP 104 | | 0 | 30 | 50 | 70 |
| Wt. % C | | 100 | 70 | 50 | 30 |
| Time, Hrs | Fluid | % C Dissolved | | | |
| 1 | SGF | 5 | 67 | 37 | 14 |
| 2 | SIF | 26 | 92 | 87 | 50 |
| 3 | SIF | 42 | | | 61 |
| 4 | SIF | 53 | | | 93 |
| 5 | SIF | 62 | | | 104 |
| 6 | SIF | 70 | | | |
| 7 | SIF | 75 | | | |

In Examples 38 to 40, the graft copolymers increased the rate of dissolution of the active ingredient, which could be advantageous in many cases.

| Example No. | | 41 | 42 | 43 | 44 | 45 |
|---|---|---|---|---|---|---|
| Wt. % SGP 502S | | 0 | 30 | 50 | 60 | 70 |
| Wt. % C | | 100 | 70 | 50 | 40 | 30 |
| Time, Hrs | Fluid | % C Dissolved | | | | |
| 1 | SGF | 5 | 85 | 12 | 13 | 9 |
| 2 | SIF | 26 | 91 | 77 | 51 | 14 |
| 3 | SIF | 42 | | 86 | 74 | 19 |
| 4 | SIF | 53 | | 95 | 91 | 24 |
| 5 | SIF | 62 | | | 97 | 30 |
| 6 | SIF | 70 | | | 100 | 35 |
| 7 | SIF | 75 | | | | -- |
| 24 | SIF | | | | | 103 |

It can be seen the tablets provide highly desirable release patterns for active ingredients.

<u>What is Claimed is:</u>

1. A pharmaceutical composition comprising a saponified starch-acrylonitrile graft copolymer and a physiologically or pharmacologically active substance.

2. A composition of claim 1 where the saponified copolymer is a starch-g-poly(acrylamide-co-sodium acrylate) graft terpolymer.

3. A composition in accordance with claim 1 or 2 where the active substance comprises from about 30 to about 90 percent by weight of the composition.

4. A composition in accordance with claims 1, 2 or 3 in dosage form.

5. A composition in accordance with claim 1, 2, 3, or 4 where the active substance is present in an amount at least sufficient for the total dosage during a treatment period.

6. A composition in accordance with any of the preceding claims in the form of a tablet.

Claims for Austria

1. A method of preparing a pharmaceutical composition comprising mixing a saponified starch-acrylonitrile graft copolymer with a physiologically or pharmacologically active substance.

2. A method of claim 1 where the saponified copolymer is a starch-g-poly(acrylamide-co-sodium acrylate) graft terpolymer.

3. A method as claimed in claim 1 or 2 where the active substance comprises from about 30 to about 90 percent by weight of the composition.

4. A method as claimed in claims 1, 2, or 3 wherein the composition is prepared in dosage unit form.

5. A method as claimed in claim 1, 2, 3 or 4 where the active substance is present in an amount at least sufficient for the total dosage during a treatment period.

6. A method as claimed in any of the preceding claims where the composition is formed into a tablet.

European Patent Office

**EUROPEAN SEARCH REPORT**

0169658

Application number

EP 85 30 4326

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | DIE PHARMAZIE, vol. 34, no. 10, 1979, pages 64-65, VEB Verlag Volk und Gesundheit, Berlin, DE; R.HÜTTENRAUCH et al.: "Pfropf-copolymere als neue Gruppe pharmazeutischer Hilfsstoffe. Einführung hochaktiver Quellstoffe" * Page 664, column 1, last paragraph; page 665, column 1, paragraph 2 * ----- | 1,2,4, 5,6 | A 61 K 47/00 |
| | | | TECHNICAL FIELDS SEARCHED (Int. Cl 4) |
| | | | A 61 K |

The present search report has been drawn up for all claims

| Place of search | Date of completion of the search | Examiner |
|---|---|---|
| THE HAGUE | 01-10-1985 | PEETERS J.C. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82